# EUROPÄISCHE PATENTANMELDUNG

(11) **EP 2 138 134 A1**
(43) Veröffentlichungstag der Anmeldung: **30.12.2009**
(21) Anmeldenummer: 09161556.7
(22) Anmeldetag: 29.05.2009
(51) Int. Cl.: A61F 2/82

(54) **Faserstrang und implantierbarer Stützkörper mit einem Faserstrang**

(30) Priorität: 25.06.2008 DE 102008002641
(71) Anmelder: Biotronik VI Patent AG, 6341 Baar (CH)
(72) Erfinder: Bertsch, Torben, 90419 Nürnberg (DE); Müller, Heinz, 91052, Erlangen (DE)
(74) Vertreter: Lindner-Vogt, Karin L.

(57) **Zusammenfassung**

Die Erfindung betrifft einen Faserstrang (10) für einen implantierbaren Stützkörper (100), der wenigstens zwei Einzelfasern (12) umfasst. Die wenigstens zwei Einzelfasern (12) sind jeweils in ihrer Längsausdehnung kürzer als eine Faserstranglängsausdehnung (14) und ihrer Querausdehnung jeweils dünner als eine Faserstrangquerausdehnung (16).

## Beschreibung

Die Erfindung betrifft einen Faserstrang und einen implantierbaren Stützkörper mit einem Faserstrang nach den Oberbegriffen der unabhängigen Ansprüche.

Heutige implantierbare Stützkörper, so genannte Stents, sind in ihren mechanischen Möglichkeiten beschränkt. So sind beispielsweise nach den bisherigen Methoden keine Strukturen umsetzbar, welche die physiologischen Bedürfnisse des umliegenden Gewebes darstellen. Radiale, longitudinale oder in Abschnitten des Implantats unterschiedlich auftretende Kräfte können bisher lediglich einzeln, aber nicht in der Gesamtheit durch ein Implantat imitiert und nachgebildet werden, weshalb Funktion und Kompatibilität eingeschränkt sind.

Viele Endoprothesen haben den Zweck, im Inneren des Körpers eines Patienten eine Stützfunktion zu übernehmen. Dementsprechend sind Endoprothesen implantierbar ausgeführt und besitzen eine Tragstruktur, die die Stützfunktion gewährleistet. Bekannt sind Implantate aus massiven metallischen Werkstoff-Strukturen. Die Wahl von Metallen als Werkstoff für die Tragstruktur eines derartigen Implantats beruht auf Erfahrungswerten aus der klassischen Mechanik und deren relativ gut beherrschten Biokompatibilität.

Metallische Stents sind in großer Zahl und verschiedenen Ausgestaltungen bekannt. So zeigt die US 2006/0212055 A1 beispielsweise verschiedene Ausgestaltungen von aus Einzelfasern bestehenden Stents. Einer der Hauptanwendungsbereiche solcher Stents ist das dauerhafte Weiten und Offenhalten von Gefäßverengungen, insbesondere von Verengungen (Stenosen) der Herzkranzgefässe. Durch den Einsatz von Stents kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents führen können.

Der Erfindung liegt die Aufgabe zugrunde, zur Verbesserung der Probleme des Standes der Technik einen Faserstrang und einen implantierbaren Stützkörper mit einem Faserstrang zu schaffen. Ebenso soll ein Verfahren zur Herstellung eines Stützkörpers angegeben werden

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Die Erfindung geht von einem Faserstrang aus, welcher wenigstens zwei Einzelfasern umfasst. Es wird vorgeschlagen, dass die wenigstens zwei Einzelfasern jeweils in ihrer Einzelfaserlängsausdehnung kürzer sind als eine Faserstranglängsausdehnung und ihrer Einzelfaserquerausdehnung jeweils dünner sind als eine Faserstrangquerausdehnung im Einsatzfall in einem aus dem Faserstrang gebildeten Produkt. Es lässt sich ein Faserstrang darstellen, der eine durch geeignete Werkstoffwahl und durch eine gewählte Verarbeitungsdichte gut steuerbare und vorhersagbare Eigenschaften aufweist. Je nach Verarbeitungsdichte kann der Faserstrang mehr oder weniger flexibel sein, mehr oder weniger mechanisch beanspruchbar, mehr oder weniger reckarm und/oder mehr oder weniger hart ausgebildet werden. Die mechanischen Eigenschaften können weiterhin auch unterschiedlich in unterschiedlichen Richtungen ausgeprägt sein. Je nach Zusammensetzung des Faserstrangs kann die chemische Stabilität gezielt eingestellt werden. Eine Korrosion und, falls gewünscht, eine Medikation kann gezielt über unterschiedliche Faserstrukturen bzw. deren Zusammensetzung eingestellt werden. Dabei sind unter der Längs- und Querausdehnung des Faserstrangs vorzugsweise die Dimensionen zu verstehen, welche bei der Weiterverarbeitung zu einem bevorzugten implantierbaren Stützkörper bzw. zu einer bevorzugten Stützstruktur der Längs- und Querausdehnung des Faserstrangs in diesem Produkt entsprechen. Aus dem Faserstrang kann ein Faserkörper gebildet werden, der durch Umformen des Faserstrangs oder durch Zusammenfügen von mehr als einem, mindestens zwei Fasersträngen gebildet wird.

Es ist vorteilhaft möglich, eine kontrollierbare und individuell anpassbare Stützstruktur zu schaffen.

Bevorzugt kann das Verhältnis von Einzelfaserlängsausdehnung zu Faserstranglängsausdehnung im Bereich zwischen 2:1 und 200000:1, vorzugsweise zwischen 10:1 und 100000:1, liegen. Bevorzugt kann das Verhältnis von Einzelfaserquerausdehnung zu Faserstrangquerausdehnung im Bereich zwischen 2:1 und 200000:1, vorzugsweise zwischen 10:1 und 100000:1, liegen. Dabei kann das Verhältnis der Längenausdehnung entkoppelt vom Verhältnis der Querausdehnung gewählt werden. Durch die vorzugsweise sehr kurzen Längen und/oder Durchmesser der Einzelfasern im Vergleich zu den Abmessungen eines aus dem Faserstrang gebildeten bevorzugten Stützkörpers können die gewünschten mechanischen Eigenschaften besonders leicht eingestellt werden. Darüber hinaus können unterschiedlichste Materialien miteinander kombiniert werden. Es kann eine große Oberfläche zur Aufnahme von Wirkstoffen in dem Faserstrang geschaffen werden. Auch kann eine biologisch wirksame Substanz bzw. Material in Faserform eingebracht werden, um lokale oder/und regionale, medizinisch positive Effekte hervorzurufen.

Gemäß einer vorteilhaften Ausgestaltung kann wenigstens eine der Einzelfasern ein Material der Gruppe Kohlenstoff, kohlenstoffhaltiges Material umfassen. Es können insbesondere Kohlefasern zum Einsatz kommen, Polymere oder organisch oder semiorganische Materialien. Semiorganische Materialien sind Materialien, die ihren Ursprung in einst lebenden Organismen haben oder daran angelehnte Strukturen besitzen.

Gemäß einer weiteren vorteilhaften Ausgestaltung kann wenigstens eine der Einzelfasern aus einem metallischen Material, umfassend wenigstens ein Mitglied / Element der Gruppe Fe, Cr, Co, Wo, Ni, Zn, Mg, Ti, Mn, Pt, Mo, Ta, Ir, Ag, Au, in kristalliner, teilkristalliner und/oder amorpher Struktur, gebildet sein. Dabei können reine Materialien und/oder Legierungen und/oder Verbindungen eines oder mehrerer der Mitglieder der Gruppe vorgesehen sein. Die Einzelfasern können z.B. aus einem amorphen Metall bzw. Legierung bestehen, etwa aus metallischen Gläsern.

Gemäß einer weiteren vorteilhaften Ausgestaltung kann wenigstens eine der Einzelfasern aus wenigstens einem keramischen Material, umfassend wenigstens ein Mitglied / Element der Gruppe Br, I, Zr, Al, N, F, Si, Ga, Ti, O, Au, Ag, in kristalliner, teilkristalliner und/oder amorpher Struktur, gebildet sein. Dabei können reine Materialien und/oder Legierungen und/oder Verbindungen eines oder mehrerer der Mitglieder der Gruppe vorgesehen sein.

Ebenso kann eine Ausgestaltung aus einer Mischung von Einzelfasern möglich sein, die aus mindestens zwei der genannten Gruppen ausgewählt sind. So lassen sich auf einfache Weise verschiedenste Materialien in Form von Einzelfasern miteinander kombinieren. Der so gebildete Faserstrang kann durch eine geeignete Kombination von Einzelfasern gewünschten Eigenschaften entsprechend optimiert werden.

Teile dieser Mischung bzw. Kombination von Einzelfasern können bereits aus medizinisch wirksamen Materialien bestehen bzw. es können medizinisch wirksame Substanzen in Einzelfasern eingearbeitet sein, und/oder Umbau- oder Abbauprodukte können medizinisch positive Wirkungen erzielen.

Der Faserstrang kann mit einem Matrixmaterial getränkt sein, welches die Einzelfasern umgeben kann. Vorzugsweise kann das Matrixmaterial wenigstens einen Stoff aus der Gruppe Wirkstoff, Polymer, mit Wirkstoff beladenem Polymer, mit Wirkstoff beladenem biodegradierbarem bzw. -absorbierbaren Polymer, umfassen, insbesondere wenigstens einen Stoff aus der Gruppe der Lipidregulatoren (Fibrate), Immunsuppressiva, der Vasodilatatoren (Sartane), der Calciumkanalblocker, Calcineurininhibitoren (Tacrolimus), der Antiflogistika (Cortison, Diclofenac), der Antünflammatorica (Imidazole), der Antiallergika, der Oligonucleoptide (dODN), der Östrogene (Genistein), der Endothelbildner (Fibrin), der Steroide, der Proteine/Peptide, der Proliferationshemmer, der Analgetika und Antirheumatika, Paclitaxel, Rapamycin, beladenen Polymernichtresorbierbarer/permanenter Polymere wie Polypropylen, Polyethylen, Polyvinylchlorid, Polyacrylate (Polyethyl- und Polymethylacrylate, Polymethylmetacrylat, Polymethyl-co-ethyl-acrylat, Ethylen/Ethylacrylat) Polytetrafluorethylen (Ethylen/Chlortrifluorethylen Copolymer, Ethylen/Tetrafluorethylen Copolymer), Polyamide (Polyamidimid, PA-11, PA-12, PA-46, PA-66), Polyetherimid, Polyethersulfon, Poly(iso)butylen, Polyvinylchlorid, Polyvinylfluorid, Polyvinylalkohol, Polyurethan, Polybuthylenterephthalat, Silikone, Polyphosphazen, Polymerschäume (z.B. aus Carbonaten, Styrolen), sowie die Copolymere und Blends der aufgezählten Klassen, bzw. der Klasse der Thermoplaste und der Elastomere im Allgemeinen, oder/und mit resorbierbaren/bioresorbierbaren/degradierbaren beladenen Polymere wie Poly-dioxanon, Polyglycolid Polycaprolacton, Polylactide [Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(I-Lactid-co-trimethylen carbonat)], Triblockcopolymere, Polysaccharide [Chitosan, Levan, Hyaluronsäure, Heparin, Dextran, Cellulose etc.], Polyhydroxyvalerat, Ethylvinylacetat, Polyethylenoxid, Polyphosphorylcholin, Fibrin, Albumin, Polyhydroxybuttersäure (ataktisch, isotaktisch, syndiotaktisch sowie deren Blends), etc.

Die Einbindung von einem oder mehreren Wirkstoffen zu einer lokalen, regionalen und/oder systemischen Therapie mittels eines aus dem Faserstrang gebildeten Produkts kann mittels Trägersubstanzen erfolgen. Als solche eignen sich degradierbare oder auch nichtdegradierbare Polymere, Fette oder andere organische Verbindungen wie z.B. Zucker, Eiweiße etc. Die Einbindung des einen Wirkstoffs oder der mehreren Wirkstoffe kann auch direkt in Form von Beschichtungen oder der Ausnutzung von Hohlräumen z.B. zwischen den Einzelfasern erfolgen. Je nach Faserzusammensetzung ist die chemische Stabilität in Form von Korrosion unterschiedliche einstellbar. Das Polymer kann günstigerweise aus wenigstens einem Monomer aus der Gruppe Lactide, Glycolide, Para-Dioxanon, Caprolacton, Trimethylencarbonat, Mischungen davon, Copolymere davon, gebildet sein. Es handelt sich vorzugsweise um biodegradierbare Stoffe, so dass aus dem Faserstrang ein biodegradierbares Produkt, etwa ein implantierbarer Stützkörper, hergestellt werden kann.

Bevorzugt können die Einzelfasern zur Bildung des Faserstrangs gedreht, geflochten und/oder gewoben sein. Sie bilden damit einen innigen Verbund mit einer einstellbar großen Oberfläche. Je nach mechanischer Verbindungsart und Einzelfasermaterial lassen sich mechanische Eigenschaften des Faserstrangs einstellen.

Vorteilhaft kann der Faserstrang zu einem Faserkörper in dreidimensionaler Form, z.B. einer Röhre, oder in flächiger Form, z.B. Band, oder auch streifenartig, z.B. als Litze, verarbeitet sein. Der z.B. als Litze ausgebildete Faserkörper kann dabei eine oder mehrere Litzen umfassen, ebenso kann z.B. das Band aus mehreren Fasersträngen oder Litzen gebildet werden oder der Faserstrang wird unmittelbar als Band oder Röhre gebildet.

Ein als Röhre ausgebildeter Faserkörper kann vorteilhaft die tragende Struktur eines implantierbaren Stützkörpers darstellen. Alternativ kann aus einer geeigneten Kombination mindestens zweier Litzen die tragende Struktur eines bevorzugten implantierbaren Stützkörpers ausgebildet werden. Dabei können auch verschiedene Litzen mit unterschiedlicher Zusammensetzung ihrer Einzelfasern, unterschiedlicher oder keiner Wirkstoffbeladung und/oder unterschiedlicher mechanischer Ausgestaltung miteinander verbunden werden.

Gemäß einer vorteilhaften Ausgestaltung kann der Faserstrang so ausgebildet sein, dass um einen Kern ein Mantel aus einem oder mehreren Litzen angeordnet ist. Der Kern kann aus einem oder mehreren Litzen ausgebildet sein. Der Mantel kann aus einem Material gebildet sein, das wenigstens einen mit einem Mantel umhüllten Kern aufweist. Mantel und/oder Kern können ebenfalls aus Litze gebildet sein.

Die Einzelfasern des Faserstrangs können z.B. durch Flechten, Weben, Knüpfen, Verdrillen und dergleichen miteinander mechanisch verbunden werden. Die Weiterverarbeitung des Faserstrangs zu einem Faserkörper kann dadurch erleichtert werden. Der Faserkörper insbesondere als Röhre, Matte oder dergleichen ausgebildet, kann nach Wunsch mit vorgegebenen Abmessungen zugeschnitten werden.

Der als Litze ausgebildete Faserkörper kann gegebenenfalls mit einem Matrixmaterial beaufschlagt sein. Dies kann auch nach Bildung der Litze erfolgen oder auch bei der Herstellung der Faserstränge. Ebenso ist denkbar, dass getränkte Litzen weiterverarbeitet werde, etwa zu einer weiteren Litze, und die fertige Litze ebenfalls mit einem Matrixmaterial beaufschlagt wird. Das Matrixmaterial für den Faserstrang und/oder den aus dem Faserstrang gebildeten Faserkörper und/oder die Litze kann wenigstens einen Stoff aus der Gruppe Wirkstoff, Polymer, mit Wirkstoff beladenem Polymer, mit Wirkstoff beladenem biodegradierbarem Polymer, umfassen. Vorzugsweise kann das Polymer aus wenigstens einem Monomer aus der Gruppe Lactide, Glycolide, Para-Dioxanon, Caprolacton, Trimethylencarbonat, Mischungen davon, Copolymere davon, gebildet sein.

Insbesondere werden bevorzugte Polymere für die Polymermatrix des erfindungsgemäßen Implantats ausgewählt aus der Gruppe:
- nichtresorbierbare / permanente Polymere:
   Polypropylen, Polyethylen, Polyvinylchlorid, Polymethylmethacrylat, Polymethylmethacrylat, Polytetrafluorethylen, Polyvinylalkohol, Polyurethan, Polybuthylenterephthalat, Silikone, Polyphosphazen sowie deren Copolymere und Blends;
- resorbierbare / bioresorbierbare / degradable Polymere:
   Polydioxanon, Polyglycolid Polycaprolacton, Polylactide (Poly-L-Lactid, Poly-D,L-Lactid, und Copolymere sowie Blends, wie etwa Poly(L-Lactid-co-glycolid), Poly(D,L-lactid-co-glycolid), Poly(L-Lactid-co-D, L-Lactid), Poly(I-Lactid-co-trimethylencarbonat, Triblockcopolymere), Polysaccharide (Chitosan, Levan, Hyaluronsäure, Heparin, Dextran, Cellulose etc.), Polyhydroxyvalerat, Ethylvinylacetat, Polyethylenoxid, Polyphosphorylcholin, Fibrin, Albumin.

Das Beaufschlagen der Litze etwa durch Tränken oder Umhüllen kann, wie gegebenenfalls bei dem Faserstrang und/oder dem Faserkörper, z.B. durch Spritzen, Tauchen und dergleichen erfolgen.

Gemäß einem weiteren Aspekt der Erfindung wird ein implantierbarer Stützkörper mit einem Faserstrang mit wenigstens einem der vorstehend beschriebenen Merkmale vorgeschlagen. Die mechanischen und die chemischen Eigenschaften des Stützkörpers können gezielt eingestellt werden. Die mechanischen Eigenschaften können durch die Verwendung des bevorzugten Faserstrangs, etwa in Form einer Röhre oder Litze, unterschiedlich in unterschiedlichen Richtungen ausgeprägt sein. Je nach Zusammensetzung des Faserstrangs kann die Stabilität des Stützkörpers gegenüber chemischen Angriffen gezielt eingestellt werden. Eine Korrosion und, falls gewünscht, eine Medikation kann gezielt über unterschiedliche Litzen oder durch innerhalb einer Litze variierende Zusammensetzungen von einem oder mehreren Wirkstoffen eingestellt werden.

Gemäß einem weiteren Aspekt der Erfindung wird ein Verfahren zur Herstellung eines implantierbaren Stützkörpers vorgeschlagen, bei dem die Schritte erfolgen
- Bereitstellen einer Litze und/oder eines Faserstrangs; und
- Zurechtschneiden der Litze und/oder des Faserstrangs
- Formen der Litze und/oder des Faserstrangs nach dem Schneiden
- Drillen, Flechten oder Weben der oben genannten Komponenten zu einer Endgeometrie.

Das Zuschneiden der Litze und/oder des Faserstrangs kann mittels Laserstrahlschneiden, Wasserstrahlschneiden oder anderen geeigneten Mitteln erfolgen.

Vorteilhaft kann die Litze und/oder der Faserstrang mit einem Matrixwerkstoff beaufschlagt werden. Dies kann durch Tränken bzw. Umhüllen etwa mittels Spritzen, Tauchen und dergleichen erfolgen.

Die Litze und der der Faserstrang kann nach dem Schneiden zu einer gewünschten Endgeometrie geformt bzw. weiterverarbeitet werden.

Die Erfindung ist nachfolgend beispielhaft, anhand von in Zeichnungen dargestellten Ausführungsbeispielen, näher erläutert. Es zeigen in schematischer Darstellung:
- Fig. 1a-c: eine Draufsicht auf einen bevorzugten streifenartigen Faserstrang (Fig. 1a), einzelne Fasern des Faserstrangs (Fig. 1b) sowie einen Ausschnitt einer Faser im Detail;
- Fig. 2a, b: einen Querschnitt eines bevorzugen Faserkörpers in Form einer aus Litze und Fasersträngen gebildeten Röhre (Fig. 2a) und ein Detail der Röhre (Fig. 2b);
- Fig. 3a-d: verschiedene Ansichten von Komponenten eines Faserstrangs mit Kern und Mantel;
- Fig. 4a, b: einen Ausschnitt ais einem aus Fasersträngen gebildeten Faserkörpers (Fig. 4a) und ein Detail des Faserkörpers (Fig. 4b);
- Fig. 5: einen bevorzugten Stützkörper aus einem bevorzugten Faserstrang.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

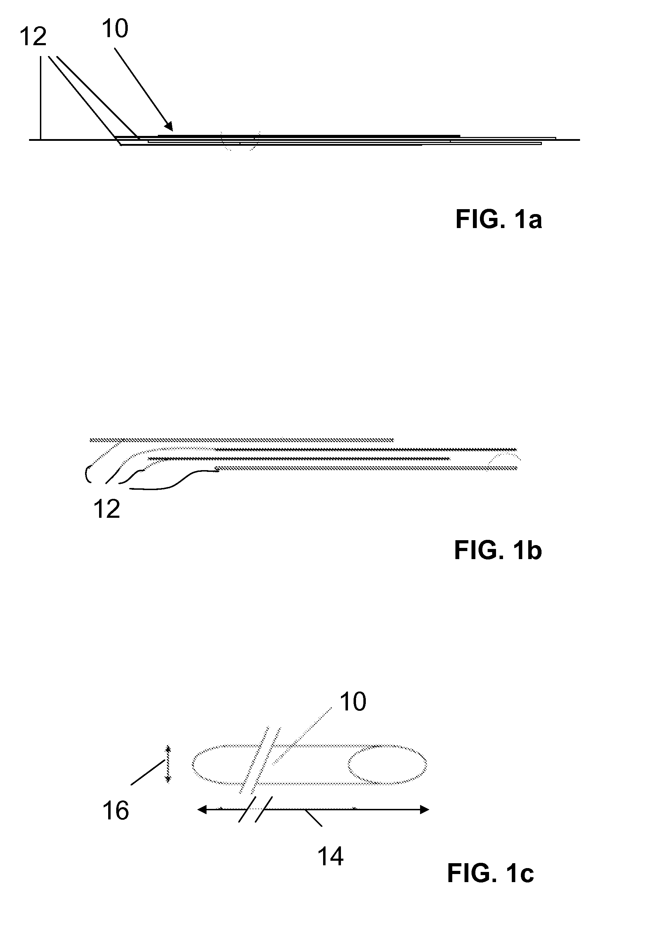

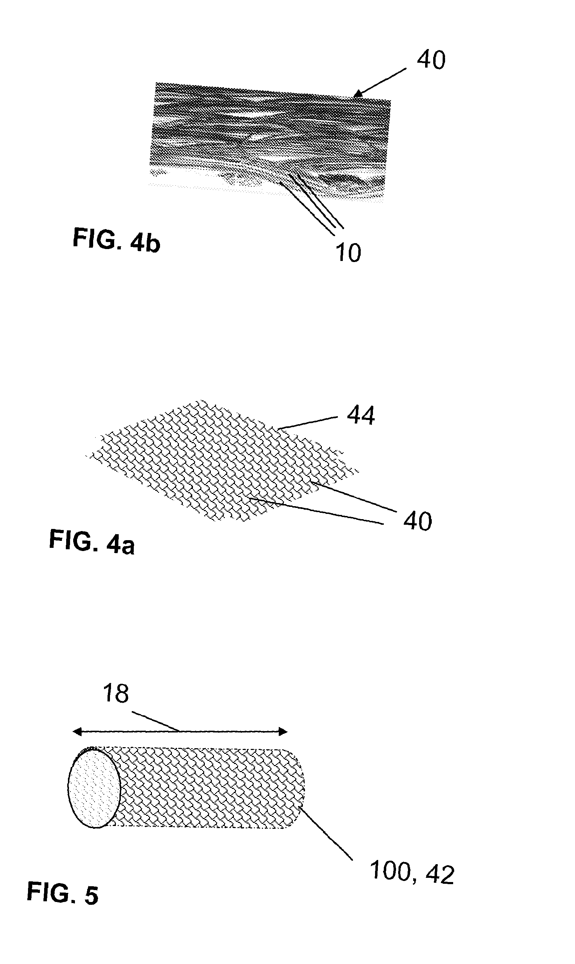

Zur Erläuterung der Erfindung zeigen die Fig. 1a einen bevorzugten Faserstrang 10, der z.B. das Ausgangsmaterial für einen bevorzugten implantierbaren Stützkörpers 100 (Fig. 5) bilden kann. Der Faserstrang 10 ist in diesem Beispiel aus einer Mehrzahl von Einzelfasern 12 gebildet, wie in Fig. 1b hervorgehoben ist.

Die Einzelfasern 12 sind in ihrer Länge jeweils erheblich kürzer als die Faserstrukturlängsausdehnung 14 und ihrem Durchmesser jeweils erheblich dünner als eine Faserstrukturquerausdehnung 16, selbst bei einem unrunden Querschnitt der Faserstruktur 10 (Fig. 1c). Die Einzelfasern 12 sind vorzugsweise dünner als die Breite und die Dicke des Faserstrangs 10. Vorteilhaft ist ein Verhältnis von Einzelfaserlängsausdehnung zu Faserstranglängsausdehnung 14 im Bereich zwischen 2:1 und 200000:1, vorzugsweise zwischen 10:1 und 100000:1, sowie ein Verhältnis von Einzelfaserquerausdehnung zu Faserstrangquerausdehnung 16 im Bereich zwischen 2:1 und 200000:1, vorzugsweise zwischen 10:1 und 100000:1.

Die Einzelfasern 12 können aus einer ersten Gruppe gebildet sein, die wenigstens ein Mitglied der Gruppe Kohlenstoff oder kohlenstoffhaltigem Material umfasst und/oder aus metallischem Material und/oder keramischem Material in reiner Form, gemischter Form sowie in kristalliner, teilkristalliner oder amorpher Struktur. Insbesondere kann wenigstens eine der Einzelfasern 12 aus einem metallischen Material umfassend wenigstens ein Mitglied/Element der zweiten Gruppe Fe, Cr, Co, Wo, Ni, Zn, Mg, Ti, Mn, Pt, Mo, Ta, Ir, Ag, Au, gebildet sein; oder wenigstens eine der Einzelfasern 12 kann aus wenigstens einem keramischen Material umfassend wenigstens ein Mitglied/Element der dritten Gruppe Br, I, Zr, Al, N, F, Si, Ga, Ti, O, Au, Ag gebildet sein. Denkbar sind auch Mischungen von Einzelfasern 12 aus zwei oder mehr der drei genannten Gruppen.

Die Einzelfasern 12 im Faserstrang 10 können mit einem Matrixmaterial 22 getränkt sein. Das Matrixmaterial kann z.B. einen Stoff aus der Gruppe Wirkstoff, Polymer, mit Wirkstoff beladenem Polymer, mit Wirkstoff beladenem biodegradierbarem Polymer, enthalten. Das Polymer kann aus wenigstens einem Monomer aus der Gruppe Lactide, Glycolide, Para-Dioxanone, Caprolacton, Trimethylencarbonat, Mischungen davon, Copolymere davon, gebildet sein. Ist das Matrixmaterial 22 mit einem Wirkstoff beladen, kann je nach gewähltem Polymer eine schnelle oder langsame Freisetzung des Wirkstoffs am Einsatzort erreicht werden. Insbesondere können bevorzugte Polymere für die Polymermatrix des erfindungsgemäßen Implantats aus der Gruppe ausgewählt werden:
- nichtresorbierbare / permanente Polymere:
   Polypropylen, Polyethylen, Polyvinylchlorid, Polymethylmethacrylat, Polymethylmethacrylat, Polytetrafluorethylen, Polyvinylalkohol, Polyurethan, Polybuthylenterephthalat, Silikone, Polyphosphazen sowie deren Copolymere und Blends;
- resorbierbare / bioresorbierbare / degradable Polymere:
   Polydioxanon, Polyglycolid Polycaprolacton, Polylactide (Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(I-Lactid-co-trimethylen carbonat, Triblockcopolymere), Polysaccharide (Chitosan, Levan, Hyaluronsäure, Heparin, Dextran, Cellulose etc.), Polyhydroxyvalerat, Ethylvinylacetat, Polyethylenoxid, Polyphosphorylcholin, Fibrin, Albumin.

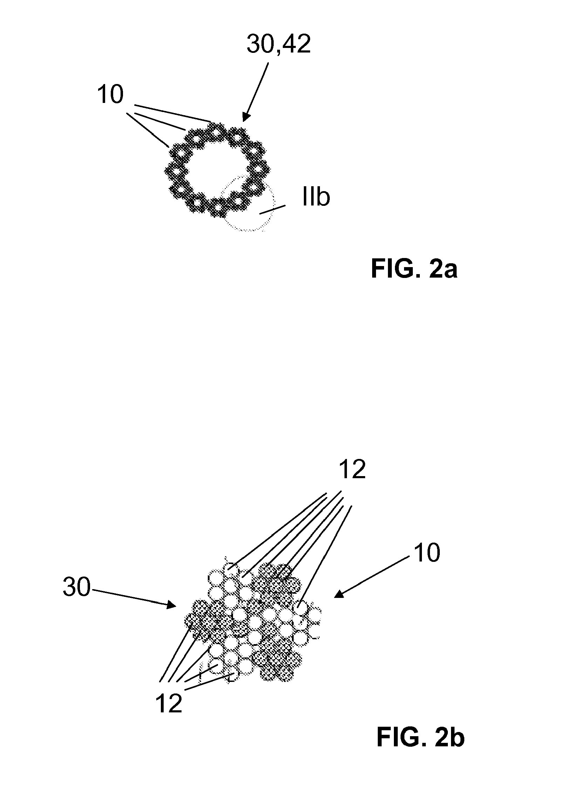

In einer günstigen Ausgestaltung der Einzelfasern 12 können diese Einzelfasern 12 aus Kohlenstoffverbindungen oder Oxid-Verbindungen, z.B. Titanoxid, bestehen, mit Durchmessern im Bereich von 0,05 µm bis 500 µm, vorzugsweise um 1 µm. Es kann aus den Einzelfasern 12 ein Faserstrang 10 und daraus ein Faserkörper, z.B. in Form einer Faserlitze 30, geflochten werden, die als Röhre 42 ausgebildet sein kann. Dies ist beispielhaft in Fig. 2a als Querschnitt durch die Röhre 42 dargestellt. Die Einzelfasern 12 können optional auch direkt zu der Röhre 42 miteinander verbunden werden.

Fig. 2b zeigt ein Detail der Hülle der Röhre 42. Die Hülle der Rühre 42 kann aus Litze 30 (ungemusterte Querschnitte) und aus Einzelfasern 12 (gemusterte Querschnitte) gebildet sein. Die Röhre 42 kann z.B. als Stützkörper eines Stents dienen und zum Einführen an ihren Bestimmungsort auf einen kleineren Durchmesser komprimiert werden. Erst am Zielort wird durch einen Aufdehn-Vorgang die endgültige mechanische Eigenschaft und Form eingestellt. Der Stützkörper kann ein Teilstück der Röhre 42 mit einer Länge 18 sein (Fig. 5), die von der Röhre 42 abgeteilt wird, etwa mit Laserschneiden. Die Röhre 42 kann mit einem Polymer, etwa einem PLGA (PLGA = Poly(lactic-co-glycolic acid)) getränkt sein, welches einen Wirkstoff z.B. mit antiinflammatorischer oder/und antiproliferativer Wirkung enthält.

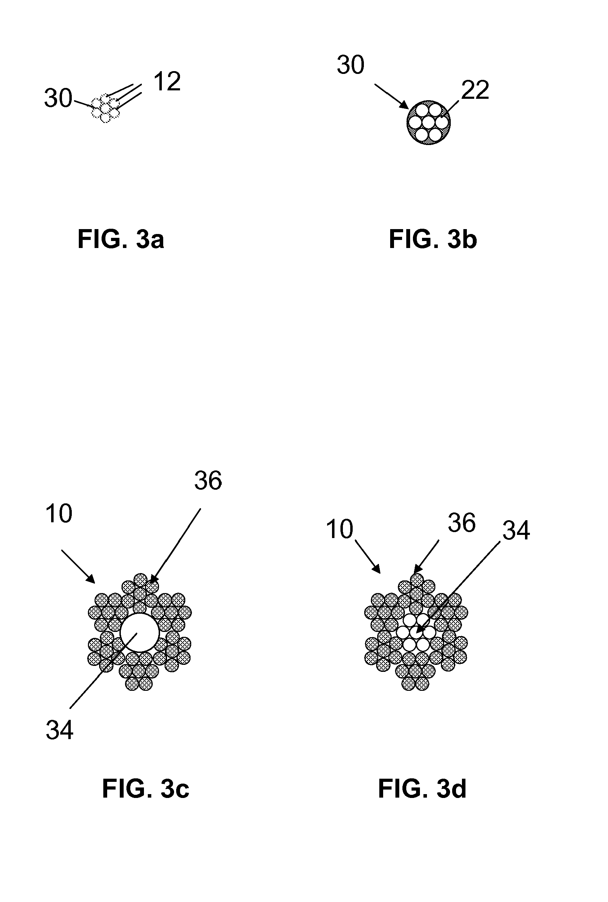

Die Fig. 3a bis 3d zeigen vorteilhafte Ausgestaltungen einer bevorzugten Litze 30 (Fig. 3a), bei der um einen Kern 34 ein Mantel 36 aus einem oder mehreren Fasersträngen 10 angeordnet ist (Fig. 3c, 3d). Der Kern 34 kann selbst aus einem oder mehreren Fasersträngen 10 bestehen (Fig. 3d), und der Mantel 36 kann aus einem Material gebildet sein, das wenigstens einen mit einem Mantel 36 umhüllten Kern 34 aufweist. Ein oder mehrere Faserstränge 10 können mit einem Matrixmaterial 22 umgeben sein (Fig. 3b).

Die Fig. 4a und 4b zeigen eine günstige Ausgestaltung eines flächigen Faserkörpers in Form eines Bandes 44 aus einer Mehrzahl von Fasersträngen 10. Aus einem solchen Band 44 kann ein oder mehrere Streifen 40 mit gewünschten Abmessungen zur Herstellung z.B. eine Stützkörpers 100 (Fig. 5) ausgeschnitten werden, der dann in eine gewünschte Geometrie gebracht wird. Ein solches Band 44 kann auch direkt aus Einzelfasern 12 gebildet werden.

Ein bevorzugter Stützkörper 100 ist in Fig. 5 als Röhre 42 dargestellt. Die Röhre 42 ist z.B. aus einer Mehrzahl von Streifen 40 aus der Matte 44 zurechtgeschnitten und mit einer gewünschten Länge und einem gewünschten Durchmesser gewirkt worden. Denkbar ist auch, das Band 44 direkt zu dem gewünschten Stützkörper 100 zu formen. Selbstverständlich kann auch eine Litze wie in den Fig. 3a-3e dargestellt ist, zu einem Stützkörper 100 verarbeitet werden. Vorteilhaft können Form, Stabilität und Wirkstoffbeladung des Stützkörpers 100 auf den vorgesehenen Einsatzzweck des Stützkörpers 100 abgestimmt werden.

Gegebenenfalls kann der so gebildete Stützkörper 100 mit einem wirkstoffbeladenen Polymer beaufschlagt, z.B. getränkt, werden. Verglichen mit der Länge 18 des Stützkörpers 100 sind die Einzelfaserlängen 16 (Fig. 1c) erheblich kürzer, und verglichen mit den Querabmessungen sind die Querabmessungen, z.B. Durchmesser, der Einzelfasern 12 erheblich dünner als etwa der Durchmesser des Stützkörpers 100.

## Patentansprüche

1. Faserstrang für einen implantierbaren Stützkörper (100), der wenigstens zwei Einzelfasern (12) umfasst, **dadurch gekennzeichnet, dass** die wenigstens zwei Einzelfasern (12) jeweils in ihrer Längsausdehnung kürzer sind als eine Faserstranglängsausdehnung (14) und ihrer Querausdehnung jeweils dünner sind als eine Faserstrangquerausdehnung (16).

2. Faserstrang nach Anspruch 1, **dadurch gekennzeichnet, dass** mehr als zwei Einzelfasern (12), insbesondere mehr als fünf Einzelfasern (12), insbesondere mehr als sechzehn Einzelfasern (12) vorgesehen sind, wobei die Einzelfasern (12) jeweils in ihrer Längsausdehnung kürzer sind als eine Faserstranglängsausdehnung (14) und ihrer Querausdehnung jeweils dünner sind als eine Faserstrangquerausdehnung (16).

3. Faserstrang nach Anspruch 1 oder 2, **dadurch gekennzeichnet, dass** das Verhältnis von Einzelfaserlängsausdehnung zu Faserstranglängsausdehnung (14) im Bereich zwischen 2:1 und 200000:1, vorzugsweise zwischen 10:1 und 100000:1, liegt und/oder dass das Verhältnis von Einzelfaserquerausdehnung zu Faserstrangquerausdehnung (16) im Bereich zwischen 2:1 und 200000:1, vorzugsweise zwischen 10:1 und 100000:1, liegt.

4. Faserstrang nach einem der vorhergehenden Ansprüche, **dadurch gekennzeichnet, dass** wenigstens eine der Einzelfasern (12) ein Material der Gruppe Kohlenstoff, kohlenstoffhaltiges Material umfasst und/oder dass wenigstens eine der Einzelfasern (12) aus einem metallischen Material umfassend wenigstens ein Mitglied der Gruppe Fe, Cr, Co, Wo, Ni, Zn, Mg, Ti, Mn, Pt, Mo, Ta, Ir, Ag, Au, in kristalliner, teilkristalliner und/oder amorpher Struktur gebildet ist und/oder dass wenigstens eine der Einzelfasern (12) aus wenigstens einem keramischen Material umfassend wenigstens ein Mitglied der Gruppe Br, I, Zr, Al, N, F, Si, Ga, Ti, O, Au, Ag, in kristalliner, teilkristalliner und/oder amorpher Struktur gebildet ist.

5. Faserstrang nach einem der vorhergehenden Ansprüche, **dadurch gekennzeichnet, dass** die Einzelfasern (12) mit einem Matrixmaterial (22) beaufschlagt sind.

6. Faserstrang nach Anspruch 5, **dadurch gekennzeichnet, dass** das Matrixmaterial (22) wenigstens einen Stoff umfasst aus der Gruppe der Lipidregulatoren (Fibrate), Immunsuppressiva, der Vasodilatatoren (Sartane), der Calciumkanalblocker, Calcineurininhibitoren (Tacrolimus), der Antiflogistika (Cortison, Diclofenac), der Antiinflammatorica (Imidazole), der Antiallergika, der Oligonucleoptide (dODN), der Östrogene (Genistein), der Endothelbildner (Fibrin), der Steroide, der Proteine/Peptide, der Proliferationshemmer, der Analgetika und Antirheumatika, Paclitaxel, Rapamycin, beladenen Polymer- nichtresorbierbarer/permanenter Polymere wie Polypropylen, Polyethylen, Polyvinylchlorid, Polyacrylate (Polyethyl- und Polymethylacrylate, Polymethylmetacrylat, Polymethyl-co-ethyl-acrylat, Ethylen/Ethylacrylat) Polytetrafluorethylen (Ethylen/Chlortrifluorethylen Copolymer, Ethylen/Tetrafluorethylen Copolymer), Polyamide (Polyamidimid, PA-11, PA-12, PA-46, PA-66), Polyetherimid, Polyethersulfon, Poly(iso)butylen, Polyvinylchlorid, -fluorid, Polyvinylalkohol, Polyurethan, Polybuthylenterephthalat, Silikone, Polyphosphazen, Polymerschäume (aus z.B. Carbonaten, Styrolen), sowie die Copolymere und Blends der aufgezählten Klassen, bzw. der Klasse der Thermoplaste und der Elastomere im Allgemeinen, oder/und mit resorbierbarer/bioresorbierbarer/degradierbarer beladenen Polymere wie Poly-dioxanon, Polyglycolid Polycaprolacton, Polylactide [Poly-L-Lactid, Poly-D,L-Lactid, und Copolymere sowie Blends wie Poly(L-Lactid-co-glycolid), Poly(D,L-lactid-co-glycolid), Poly(L-Lactid-co-D,L-Lactid), Poly(I-Lactid-co-trimethylen carbonat)], Triblockcopolymere, Polysaccharide [Chitosan, Levan, Hyaluronsäure, Heparin, Dextran, Cellulose], Polyhydroxyvalerat, Ethylvinylacetat, Polyethylenoxid, Polyphosphorylcholin, Fibrin, Albumin, Polyhydroxybuttersäure (ataktisch, isotaktisch, syndiotaktisch sowie deren Blends).

7. Faserstrang nach Anspruch 6, **dadurch gekennzeichnet, dass** das Polymer aus wenigstens einem Monomer aus der Gruppe Lactide, Glycolide, Para-Dioxanone, Caprolactone, Trimethylencarbonat, Caprolacton, Mischungen davon, Copolymere davon, gebildet ist.

8. Faserstrang nach einem der vorhergehenden Ansprüche, **dadurch gekennzeichnet, dass** die Einzelfasern (12) durch Drehen, Flechten und/oder Weben miteinander verbunden sind, durch eine Ausgestaltung als dreidimensionale Struktur z.B. als Röhre (42), geschlossene oder mindestens einseitig geöffnete Schalenstruktur oder Stabstruktur (44).

9. Faserstrang nach einem der vorhergehenden Ansprüche, **dadurch gekennzeichnet, dass** die Einzelfasern (12) und/oder zumindest Teile des Faserstrangs (10) degradierbar oder absorbierbar sind.

10. Faserkörper mit wenigstens einem Faserstrang (10) nach einem der vorhergehenden Ansprüche, **gekennzeichnet durch** eine Ausgestaltung als Litze (30), Band (40), Röhre (42) oder Matte (44) aus einer Mehrzahl von Fasersträngen (10).

11. Faserkörper nach Anspruch 10, **dadurch gekennzeichnet, dass** wenigstens zwei Litzen (30) miteinander zu einer mechanisch tragenden Struktur, insbesondere zu einem implantierbaren Stützkörper, ausgebildet sind und, dass um einen Kern (34) ein Mantel (36) aus einem oder mehreren Fasersträngen (10) angeordnet ist und/oder dass der Kern (32) aus einem oder mehreren Fasersträngen (10) ausgebildet ist und/oder dass der Mantel (36) aus einem Faserstrang-Material gebildet ist, das wenigstens einen mit einem Mantel (36) umhüllten Kern (34) aufweist.

12. Faserkörper nach Anspruche 10 oder 11, **dadurch gekennzeichnet, dass** die Faserstränge (10) mit einem Matrixmaterial (22) beaufschlagt sind und, dass das Matrixmaterial (22) wenigstens einen Stoff aus der Gruppe Wirkstoff, Polymer, mit Wirkstoff beladenem Polymer, mit Wirkstoff beladenem biodegradierbarem Polymer, umfasst.

13. Faserkörper nach Anspruch 13, **dadurch gekennzeichnet, dass** das Polymer aus wenigstens einem Monomer aus der Gruppe Lactide, Glycolide, Para-Dioxanone, Caprolactone, Trimethylencarbonat, Caprolacton, Mischungen davon, Copolymere davon, gebildet ist.

14. Faserkörper nach einem der Ansprüche 10 bis 13, **dadurch gekennzeichnet, dass** die Einzelfasern oder aber zumindest Teile der Faserstranglitze degradierbar oder absorbierbar sind.

15. Implantierbarer Stützkörper (100) aus wenigstens einem Faserstrang (10) nach einem der Ansprüche 1 bis 9 und/oder einer Litze (30) nach einem der Ansprüche 10 bis 12.

16. Verfahren zur Herstellung eines implantierbaren Stützkörpers (100) nach Anspruch 15, **gekennzeichnet durch**
- Bereitstellen der Litze (30) und/oder eines Faserstrangs (10);
- Zurechtschneiden der Litze (30) und/oder eines Faserstrangs (10)
- Formen der Litze (30) und/oder des Faserstrangs (10) nach dem
- Drillen, Flechten oder Weben der Litze (30) und/oder des Faserstrangs (10) zu einer Endgeometrie und
- Tränken der Litze (30) und/oder des Faserstrangs (10) mit einem Matrixwerkstoff (22).
